# EUROPEAN PATENT SPECIFICATION

(11) **EP 1 492 549 B1**
(45) Date of publication and mention of the grant of the patent: **11.05.2016**
(21) Application number: 02725132.1
(22) Date of filing: 13.03.2002
(51) Int. Cl.: A61K 35/74, A61P 13/12

(54) **COMPOSITIONS AND METHODS FOR AUGMENTING KIDNEY FUNCTION**
ZUSAMMENSETZUNGEN UND VERFAHREN ZUR VERSTÄRKUNG DER NIERENFUNKTION
COMPOSITIONS ET PROCEDES POUR AUGMENTER LES FONCTIONS RENALES

(43) Date of publication of application: 05.01.2005
(73) Proprietor: Kibow Biotech, Inc., Philadelpha, Pennsylvania 19104 (US)
(72) Inventor: RANGANATHAN, Natarajan, Broomall, PA 19008 (US); DICKSTEIN, Jack, Huntingdon Valley, PA 19006 (US); MEHTA, Raj, King of Prussia, PA 19406 (US)
(74) Representative: Dunleavy, Kevin James
(86) International application number: PCT/US2002/007554
(87) International publication number: WO 2003/088984

(56) References cited:
- EP-A- 0 904 784
- EP-A- 1 243 273
- WO-A-02/091833
- WO-A2-00/71138
- WO-A2-00/71139
- WO-A2-99/49877
- DE-U1- 20 202 562
- US-A- 4 022 883
- US-A- 4 218 541
- US-A- 4 970 153
- US-A- 5 116 737
- FRIEDMAN, ELI A. [REPRINT AUTHOR] ET AL: "Gut-based uremia therapy: Simultaneous removal of urea, creatinine, and uric acid." JOURNAL OF THE AMERICAN SOCIETY OF NEPHROLOGY, (SEPTEMBER, 2001) VOL. 12, NO. PROGRAM AND ABSTRACT ISSUE, PP. 71A-72A. PRINT. MEETING INFO.: ASN (AMERICAN SOCIETY OF NEPHROLOGY)/ISN (INTERNATIONAL SOCIETY OF NEPHROLOGY) WORLD CONGRESS OF NEPHROLOGY., September 2001 (2001-09), XP008051527

## Description

### FIELD OF THE INVENTION

The present invention relates to compositions and the use thereof in methods which reduce the concentration of nitrogenous wastes. The compositions may be in the form of a food, dietary supplement, medical food or drug.

### BACKGROUND OF THE INVENTION

One of the main functions of the normal, healthy kidney, besides its regulatory, endocrine, and metabolic functions, is the disposal of waste products. Any impairment of excretory function can lead to the accumulation of a variety of nitrogenous waste products including, urea, creatinine and uric acid. High concentrations of waste products in the blood stream can exacerbate renal failure and promote kidney stones. Moreover, nitrogenous solutes in the circulating blood promote osmotic diffusion into the lumen because of the concentration gradient across the intestinal wall. This diffusion mechanism led to the concept of oral sorbents to augment gut-based clearance of nitrogenous waste products. Sorbents or microbes have demonstrated their ability to remove various compounds and nitrogenous wastes within the large bowel.

Urea-specific sorbents such as synthetic polymers and modified polysaccharides have been evaluated for the removal of urea and other nitrogenous wastes via the gut. Other sorbents such as oxidized starch, activated charcoal, and carob flour have also been investigated for the *in vivo* elimination of uremic toxins with some success. Prakash & Chang ((1996) Nature Medicine 2:883-88) demonstrated that microencapsulated, genetically-engineered *E. coli* DH5 are effective in removing urea and ammonia in an *in vitro* system. The same researchers obtained similar results in oral administration of *E. coli* DH5 cells in a uremic rat animal model. Bliss, et al. ((1996) Am. J. Clin. Nutr. 63:392-398) have demonstrated that supplemental gum arabic fiber increases fecal nitrogen excretion and lowers urea nitrogen concentration in chronic renal failure patients consuming a low protein diet. Reinhart, et al. ((1998) Rec. Adv. In Canine and Feline Nutr. Iams Nutrition Symposium Proceedings. Vol. II:395-404) found that canine renal patients fed a diet containing a fermentable fiber blend improved clinical end-stage renal disease status, suggesting that specific nutritional alteration allows repartitioning of nitrogen excretion away from the kidney and into the feces by colonic fermentation or additional bacterial growth.

Prebiotic components are food ingredients that enhance the actions of probiotic components in the digestive tract. In this synergistic or synbiotic relationship a probiotic component, such as *Bifidobacteria,* metabolizes undigested carbohydrates, such as dietary fibers, oligosaccharides, etc., to produce short-chain fatty acids such as acetate, propionate and butyrate. These short-chain fatty acids may promote intestinal cell growth, enhance water and mineral absorption, and prevent yeast, mold, and pathogenic bacterial growth. In addition, probiotic components may antagonize pathogens directly through production of antimicrobial and antibacterial compounds such as cytokines and butyric acid (De Vuyst and Vandamme. Antimicrobial potential of lactic acid bacteria. *In:* De Vuyst L, Vandamme EL, eds. Bacteriocins of lactic acid bacteria. Glasgow, United Kingdom: Blackie Academic and Professional; 1994:91-142; Dodd and Gasson. Bacteriocins of lactic acid bacteria. In: Gasson MJ, de Vos WM, eds. Genetics and biotechnology of lactic acid bacteria. Glasgow, United Kingdom: Blackie Academic and Professional; 1994:211-51; Kailasapathy and Chin (2000) Immunol. Cell. Biol. 78(1):80-8), reduce gut pH by stimulating the lactic acid-producing microflora (Langhendries, et al. (1995) J. Pediatr. Gastroenterol. Nutr. 21:177-81), compete for binding and receptor sites that pathogens occupy (Kailasapathy and Chin (2000) Immunol. Cell. Biol. 78(1):80-8; Fujiwara et al., (1997) Appl. Environ. Microbiol. 63:506-12), improve immune function and stimulate immunomodulatory cells (Isolauri et al. (1991) Pediatrics 88:90-97; Isolauri et al. (1995) Vaccine 13:310-312; Rolfe (2000) J. Nufr. 130(2S):3965-4025), compete with pathogens for available nutrients and other growth factors (Rolfe (2000) J. Nufr. 130(2S):396S-402S), or produce lactase which aids in lactose digestion.

WO 99/49877 A2 discloses a composition including at least one probiotic (e.g. *Bacillus coagulans, Bacillus brevis, Lactobacillus*) and at least one prebiotic such as a bifidogenic oligosaccharide.

WO 00/71138 A2 discloses a composition including at least one probiotic (e.g. *Lactobacillus fermentum, Bifidobacterium*) and at least one prebiotic such as inulin and use of compositions containing probiotics and prebiotics for prevention of urinary tract infections in infants.

WO 00/71139 A2 discloses a composition including at least one probiotic (e.g. *Lactobacillus fermentum, Bifidobacterium*) and at least one prebiotic such as inulin.

WO 02/091833 A2, published on 21 November 2002, discloses a composition including at least one probiotic (e.g. *Bacillus* species, *Lactobacillus*) and at least one prebiotic such as non-starch polysaccharides and the use of such compositions to treat renal failure/insufficiency as well as to remove excess nitrogenous waste products.

EP-A-1243273, published on 25 September 2002, discloses a composition including at least one probiotic (e.g. *Bacillus* species, *Lactobacillus*) and at least one prebiotic such as inulin and oligosaccharides for use in therapy.

The present invention combines the beneficial effects of prebiotic and probiotic components to into a synbiotic product that effectively reduces nitrogenous wastes.

### SUMMARY OF THE INVENTION

An object of the present invention is to provide an enteric-coated composition comprising prebiotic and probiotic components, wherein the probiotic component includes a mixed culture of bacteria selected from *Streptococcus, Lactobacillus, Bacillus,* and *Bifidobacteria* microorganisms, wherein the bacteria are provided at a concentration of from 10¹⁰ cfu/g to 10¹² cfu/g or from 10¹⁰ cfu/mL to 10¹² cfu/mL. This composition can be added to a food product, dietary supplement, medical food or pharmaceutical product which, upon ingestion, will promote a healthy intestinal microenvironment and assist in the elimination of nitrogenous waste products that can build up in concentration in the circulating blood. Increased concentrations of the wastes are known to exert a negative impact on an individual's physiology and contribute to a decreased sense of well being and general malaise. Methods for reducing nitrogenous waste products using these products are also provided.

In addition, enteric-coated compositions comprising a prebiotic and a probiotic containing at least one *Streptococcus* microorganism for use in methods of treating renal failure are provided.

### DETAILED DESCRIPTION OF PREFERRED EMBODIMENTS

Nitrogenous waste products accumulating in the blood stream have detrimental effects on health. Removal of nitrogenous wastes by diverting them into the colon is a viable approach to decrease the negative impact that waste product accumulation has on an individual's physiology. The present invention combines the properties of probiotic and prebiotic components into a synbiotic product to effectively reduce the blood concentration of nitrogenous waste products and has the added beneficial effect of promoting the growth of desirable intestinal microflora.

A probiotic component of the present invention refers to a mixed culture of live or freeze-dried microorganisms which, when applied to man or animal, beneficially affects the host by improving the properties of the indigenous microflora, such as bifidobacterium organisms that metabolize undigested carbohydrates and are beneficial to an individual. Probiotic components of the present invention are selected for their ability to exert a beneficial effect on the host, survive transit through the intestinal tract, may adhere to intestinal epithelial cell lining, produce anti-microbial substances towards pathogens and/or stabilize the intestinal microflora. Furthermore, the probiotic component must have a good shelf-life. Synbiotic products of the present invention should contain a large number of viable cells at the time of consumption, and be non-pathogenic and nontoxic. Examples of probiotic components include, but are not limited to, *Bifidobacterium* spp. (e.g., *bifidum, longum, infantis*), *Lactobacillus* spp. (e.g., *bulgaricus, acidophilus, lactis, helveticus, casei, reuteri, delbrueckii), Streptococcus* spp. (e.g., *thermophilus, diacetilactis, cremoris, durans, faecalis), Saccharomyces* spp. (e.g., *pombe, boulardii*), *Leuconostoc* spp. (e.g., *citrovorum, dextranicum*) and *Bacillus* sp. (e.g., *pasteurii*)*.* In a preferred embodiment, the probiotic component comprises one or more micoorganisms from the genera *Bifidobacterium* or *Lactobacillus.*

Microorganisms also useful in the invention are those that have the ability, either through natural selection or by genetic manipulation, to catabolize various nitrogenous compounds, including but not limited to urea, creatinine, uric acid and ammonia. An example is a microorganism having an elevated level of urease secretion. Elevated levels of secretion can be obtained by inserting the gene encoding urease into a prokaryotic microorganism such as *Lactobacillus, Bacillus,* or *Bifidobacterium,* or a eukaryotic microorganism such as *Saccharomyces.* The urease gene may be under the regulatory control of an inducible or constitutive promoter. Promoters most commonly used in recombinant prokaryotic expression vectors include the beta-lactamase (penicillinase) and lactose promoter systems (Chang et al. (1978) Nature 275:615; and Goeddel et al. (1979), Nature 281:544, a tryptophan (trp) promoter system (Goeddel et al. (1980) Nucleic Acids Res. 8:4057 and EPO App. Publ. No. 36,776) and the tac promoter (H. De Boer et al. (1983) Proc. Natl. Acad. Sci. USA 80:21). While these are commonly used, other microbial promoters are suitable. Details concerning nucleotide sequences of many have been published, enabling a skilled worker to operably-ligate them to DNA encoding the protein in plasmid or viral vectors (Siebenlist et al. (1980) Cell 20:269). The promoter and Shine-Dalgarno sequence (for prokaryotic host expression) are operably-linked to the DNA encoding the desired protein, i.e., they are positioned so as to promote transcription of the protein messenger RNA from the DNA.

Eukaryotic microbes such as yeast cultures may be transformed with suitable protein-encoding vectors. *See e.g.,* U.S. Patent No. 4,745,057. *Saccharomyces cerevisiae* is the most commonly used among lower eukaryotic host microorganisms, although a number of other strains are commonly available. Yeast vectors may contain an origin of replication from the 2 micron yeast plasmid or anautonomously replicating sequence (ARS), a promoter, DNA encoding the desired protein, sequences for polyadenylation and transcription termination, and a selection gene. An exemplary plasmid is YRp7, (Stinchcomb et al. (1979) Nature 282:39; Kingsman et al. (1979) Gene 7:141; Tschemper et al. (1980) Gene 10:157). This plasmid contains the trp1 gene, which provides a selection marker for a mutant strain of yeast lacking the ability to grow in tryptophan, for example ATCC No. 44076 or PEP4-1 (Jones (1977) Genetics 85:12). The presence of the trp1 lesion in the yeast host cell genome then provides an effective environment for detecting transformation by growth in the absence of tryptophan.

Suitable promoting sequences in yeast vectors include the promoters for metallothionein, 3-phospho-glycerate kinase (Hitzeman et al. (1980) J. Biol. Chem. 255:2073) or other glycolytic enzymes (Hess et al. (1968) J. Adv. Enzyme Reg. 7:149; Holland et al. (1978) Biochemistry 17:4900), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are further described in R. Hitzeman et al., EPO Publn. No. 73,657.

As will be understood by those of skill in the art, expression vectors containing polynucleotides which encode urease may be designed to contain signal sequences which direct secretion of urease through a prokaryotic or eukaryotic cell membrane.

Transforming the microorganisms as defined herein, describes a process by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the type of host cell being transformed and may include, but is not limited to, viral infection, electroporation, heat shock, lipofection, and particle bombardment. Such "transformed" cells include stably-transformed cells in which the inserted DNA is capable of replication either as an autonomously replicating plasmid or as part of the host chromosome. They also include cells which transiently express the inserted DNA or RNA for limited periods of time

A resulting transformed strain is selected for high levels of urease secretion by growing transformed strains on high levels of urea. Levels of urease secretion may also be detected using standard enzymatic assays. Transformed strains may be sequentially subcultured on increasingly levels of urea to enhance urease secretion. In a preferred embodiment the urease-secreting microorganism is *Bacillus pasteurii.* In another preferred embodiment the urease-secreting microorganism is *Saccharomyces pombe.*

The probiotic component or urease-secreting microorganism comprises an additive to a food product, such as a health bar, health drink, yogurt, or dahi, or to an enterically coated tablet, capsule, powder, soft gel, gelcap, or liquid. Accordingly, in a preferred embodiment of the present invention, the probiotic component or urease-secreting microorganism is included at a concentration of 10¹⁰ cfu/mL, 10¹¹ cfu/mL, or 10¹² cfu/mL when added as a liquid or 10¹⁰ cfu/g, 10¹¹ cfu/g, or 10¹² cfu/g when added as a solid.

A prebiotic component of the present invention refers to a non-digestive food that beneficially affects the host by selectively stimulating the growth and/or activity of one or more non-pathogenic bacteria in the colon. Prebiotic components of the present invention are considered to have anti-carcinogenic, anti-microbial, hypolipidemic and glucose modulatory activities. They may also improve mineral absorption and balance. Furthermore, bacteria belonging to the *Bifidobacterium* and *Lactobacillus* families are stimulated by the presence of the prebiotic component and proliferate. Pharmacokinetically, the prebiotic components reach the colon largely intact. In a preferred embodiment the prebiotic components are an oligosaccharide (e.g., fructooligosaccharide or inulin, isomaltose oligosaccharide, transgalacto-oligosaccharide, xylo-oligosaccharide, or soyoligosaccharide) or a pyrodextrin (e.g., arabinogalactan, lactilol, lactosucrose, or lactulose). The amount of prebiotic component added to the probiotic component is 100 milligrams per serving, 500 milligrams per serving, 1 gram per serving, 5 grams per serving or 10 grams per serving. In a preferred embodiment the prebiotic component is not less than 100 milligrams and not more than 10 grams per serving.

A synbiotic product combining the beneficial properties of probiotic and prebiotic components may include a food product, dietary supplement, comestible medical food or pharmaceutical product. In the context of the present invention, "synbiotic" refers to a mixture of probiotic and prebiotic components to promote health enhancing effects (Gibson and Roberfroid (1995) J. Nutr. 125:1401-1412). The ingestion of said synbiotic product reduces the blood concentration of nitrogenous waste products that accumulate in the circulating blood stream. These waste products of the present invention may be of an endogenous origin such as normal or abnormal metabolic routes or bacterial putrefaction. Furthermore, the waste products may be of an exogenous origin as in dietary intake of proteins and amino acids. Furthermore, repeated ingestion of the synbiotic product will have a highly beneficial effect upon the intestinal microflora by localization and colonization in the large intestine of microbes known to promote a healthy intestinal microenvironment.

A food product includes, but is not limited to, a health bar, health drink, yogurt, or dahi. For example, a yogurt food product can be prepared from one gallon of commercially available whole, homogenized, pasteurized milk which is heated to boiling and quickly allowed to cool to approximately 45°C. To this is added approximately one ounce of yogurt starter culture containing lactic acid bacteria of the genus *Lactobacillus, Bacillus* or *Bifidobacteria.* The mixture is mixed well and allowed to ferment at 37°C for 10 to 12 hours. Whole fruit additives, flavoring, sweetening agents, binders, or other additives can be combined and added to the yogurt to obtain a product of desired consistency or to suit the palette of the prospective consumer. In a preferred embodiment of the present invention, a food product comprises components to meet the special dietary needs of individuals with renal insufficiency.

Health bars may be prepared by combining various excipients, such as binders, additives, flavorings, colorants and the like, along with prebiotic and probiotic components, and mixing to a plastic mass consistency. The mass is then either extruded or molded to form "candy bar" shapes that are then dried or allowed to solidify to form the final product.

Various additives may be incorporated into the present compositions. Optional additives of the present composition include, without limitation, starches, sugars, fats, antioxidants, amino acids, proteins, nucleic acids, electrolytes, vitamins, derivatives thereof or combinations thereof. In a preferred embodiment, an additive of the synbiotic product is carob flour, for example, locust bean gum. In another preferred embodiment, an additive is a mushroom extract from *Agaricus bisporus.*

Flavors which may optionally be added to the present compositions are those well known in the pharmaceutical art. Examples include, but are not limited to, synthetic flavor oils, and/or oils from plants leaves, flowers, fruits and so forth, and combinations thereof are useful. Examples of flavor oils include, but are not limited to, spearmint oil, peppermint oil, cinnamon oil, and oil of wintergreen (methylsalicylate). Also useful are artificial, natural or synthetic fruit flavors such as citrus oils including lemon, orange, grape, lime, and grapefruit, and fruit essences including apple, strawberry, cherry, pineapple and so forth.

Sweetening agents may be selected from a wide range of materials such as water-soluble sweetening agents, water-soluble artificial sweeteners, and dipeptide based sweeteners, including salts thereof and mixtures thereof, without limitation.

Binders may be selected from a wide range of materials such as hydroxypropylmethylcellulose, ethylcellulose, or other suitable cellulose derivatives, povidone, acrylic and methacrylic acid co-polymers, pharmaceutical glaze, gums, milk derivatives, such as whey, starches, and derivatives, as well as other conventional binders well known to persons skilled in the art. Examples of solvents include, but are not limited to, water, ethanol, isopropyl alcohol, methylene chloride or mixtures and combinations thereof. Examples of bulking substances include, but are not limited to, sugar, lactose, gelatin, starch, and silicon dioxide.

A dietary supplement of the present invention can be prepared from lactic acid bacteria of the genus *Lactobacillus, Bacillus* or *Bifidobacteria,* and a urease-secreting microorganism. The microorganisms are aseptically freeze-dried and the processed bulk bacteria are then prepared as enterically coated capsules according to the method of Kim et al ((1988) J. Indust. Microbiol. 3:253-257). Eighty to 250 mg of the freeze-dried microorganism is contained in each capsule that is enterically coated with hydroxy-propylmethyl cellulose phthalate by spraying over a fluidized bed of capsules. The resulting dietary supplement has a low surface area, is relatively non-porous and can protect the contents therein from low pH as is found in the gastric environment for several hours, and will release the contents into the bowel wherein the pH is relatively neutral or slightly alkaline.

In addition to the aforementioned controlled release formulation, there a number of controlled release formulations that are developed preferably for oral administration. These include, but are not limited to, osmotic pressure-controlled gastrointestinal delivery systems; hydrodynamic pressure-controlled gastrointestinal delivery systems; membrane permeation-controlled gastrointestinal delivery systems, which include microporous membrane permeation-controlled gastrointestinal delivery devices; gastric fluid-resistant intestine targeted controlled-release gastrointestinal delivery devices; gel diffusion-controlled gastrointestinal delivery systems; and ion-exchange-controlled gastrointestinal delivery systems, which include cationic and anionic drugs. Additional information regarding controlled release drug delivery systems may be found in Yie W. Chien, Novel Drug Delivery Systems, 1992 (Marcel Dekker, Inc.). Some of these formulations will now be discussed in more detail. Enteric coatings are applied to tablets to prevent the release of drugs in the stomach either to reduce the risk of unpleasant side effects or to maintain the stability of the drug which might otherwise be subject to degradation of expose to the gastric environment. Most polymers that are used for this purpose are polyacids that function by virtue or the fact that their solubility in aqueous medium is pH-dependent, and they require conditions with a pH higher then normally encountered in the stomach. One preferable type of oral controlled release structure is enteric coating of a solid or liquid dosage form. Enteric coatings promote the compounds remaining physically incorporated in the dosage form for a specified period when exposed to gastric juice. Yet the enteric coatings are designed to disintegrate in intestinal fluid for ready absorption. Delay of absorption is dependent on the rate of transfer through the gastrointestinal tract, and so the rate of gastric emptying is an important factor. Some investigators have reported that a multiple-unit type dosage form, such as granules, may be superior to a single-unit type.

Typical enteric coating agents include, but are not limited to, hydroxypropylmethylcellulose phthalate, methacryclic acid-methacrylic acid ester copolymer, polyvinyl acetate-phthalate and cellulose acetate phthalate (Hasegawa, (1985) Chem. Pharm. Bull. 33:1615-1619). Various enteric coating materials may be selected on the basis of testing to achieve an enteric-coated dosage form designed ab initio to have a preferable combination of dissolution time, coating thicknesses and diametral crushing strength. (Porter et al. (1970) J. Pharm. Pharmacol. 22:42p).

A medical food can be prepared by combining rolled oats, dehydrated apples, honey, inulin, carob flour, cinnamon, sugar, vanilla extract, and lyophilized cultures of *L. sporogenes, L. acidophilus* and *L. fermentum* (10¹⁰ cfu each). These ingredients are mixed in appropriate proportions and formed into a rectangular bar approximately 12.5 to 15 centimeters in length, 3 to 4 centimeters in width and 1 centimeter in height and placed into a sterile vacuum oven for 12 to 24 hours to obtain an edible food product of the desired consistency. A pharmaceutical product for persons suffering from renal insufficiency can be prepared by aseptically freeze-drying urease-secreting microorganisms. The processed bulk microorganisms are then prepared as enterically coated capsules according to the method of Kim et al ((1988) J. Indust. Microbiol. 3:253-257), for example, 80 mg to 1 g of the freeze-dried microorganism is contained in each capsule that is enterically coated with hydroxy-propylmethyl cellulose phthalate by spraying over a fluidized bed of capsules. The resulting pharmaceutical product has a low surface area, is relatively non-porous and can protect the contents therein from low pH as is found in the gastric environment for several hours, and will release the contents into the bowel wherein the pH is relatively neutral or slightly alkaline.

## Claims

1. An enteric-coated composition including at least one probiotic component and at least one prebiotic component, wherein the probiotic component includes a mixed culture of bacteria selected from *Streptococcus, Lactobacillus, Bacillus* and *Bifidobacteria* microorganisms, wherein the bacteria are provided at a concentration of from 10¹⁰ cfu/g to 10¹² cfu/g or from 10¹⁰ cfu/mL to 10¹² cfu/mL.

2. The composition of claim 1, wherein the probiotic component includes a *Lactobacillus* or *Bifidobacteria* microorganism.

3. The composition of any one of claims 1-2, wherein the prebiotic component includes an oligosaccharide or a pyrodextrin.

4. The composition of any one of claims 1-3, wherein said *Streptococcus* microorganism is selected from *Streptococcus thermophilus, Streptococcus diacetiliactis, Streptococcus cremoris, Streptococcus durans and Streptococcus faecalis.*

5. The composition of any one of claims 1-4, wherein the *Streptococcus* microorganism is *Streptococcus thermophilus.*

6. The composition of any one of claims 1-5, wherein the *Lactobacillus* microorganism is selected from *Lactobacillus bulgaricus, Lactobacillus acidophilus, Lactobacillus lactis, Lactobacillus helveticus, Lactobacillus casei, Lactobacillus reuteri,* and *Lactobacillus delbrueckii.*

7. The composition of claim 6, wherein the *Lactobacillus* microorganism is *Lactobacillus acidophilus.*

8. The composition of any one of claims 1-7, wherein the *Bifidobacteria* microorganism is selected from *Bifidobacterium bifidum, Bifidobacterium longum,* and *Bifidobacterium infantis.*

9. The composition of claim 8 wherein the *Bifidobacteria* microorganism is *Bifidobacterium longum.*

10. The composition of any one of claims 1-9, wherein the probiotic component includes more than one *Lactobacillus* or *Bifidobacteria* microorganism.

11. The composition of any one of claims 1-9, wherein the probiotic component includes a *Lactobacillus* microorganism.

12. The composition of any one of claims 1-9, wherein the probiotic component includes a *Bifidobacteria* microorganism.

13. A composition as claimed in any one of claims 1-12, for use in reducing the blood concentration of nitrogenous waste products that accumulate in the circulating blood stream.

14. A composition as claimed in any one of claims 1-12, for use in the treatment of renal failure.

15. Use of a composition as claimed in any one of claims 1-12, for the manufacture of a medicament for treatment of renal failure.

## Patentansprüche

1. Eine enterisch beschichtete Zusammensetzung umfassend mindestens eine probiotische Komponente und mindestens eine präbiotische Komponente, wobei die probiotische Komponente eine gemischte Bakterienkultur umfasst, ausgewählt unter *Streptococcus-, Lactobacillus-, Bacillus-* und *Bifidobacterium-*Mikroorganismen, wobei die Bakterien in einer Konzentration von 10¹⁰ KbE/g bis 10¹² KbE/g oder von 10¹⁰ KbE/ml bis 10¹² KbE/ml bereitgestellt werden.

2. Die Zusammensetzung gemäß Anspruch 1, wobei die probiotische Komponente einen *Lactobacillus-* oder *Bifidobacterium-*Mikroorganismus umfasst.

3. Die Zusammensetzung gemäß irgendeinem der Ansprüche 1-2, wobei die präbiotische Komponente ein Oligosaccharid oder ein Pyrodextrin umfasst.

4. Die Zusammensetzung gemäß irgendeinem der Ansprüche 1-3, wobei besagter *Streptococcus*-Mikroorganismus ausgewählt wird unter *Streptococcus thermophilus*, *Streptococcus diacetiliactis, Streptococcus cremoris, Streptococcus durans* und *Streptococcus faecalis*.

5. Die Zusammensetzung gemäß irgendeinem der Ansprüche 1-4, wobei der *Streptococcus*-Mikroorganismus *Streptococcus thermophilus* ist.

6. Die Zusammensetzung gemäß irgendeinem der Ansprüche 1-5, wobei der *Lactobacillus*-Mikroorganismus ausgewählt wird unter *Lactobacillus bulgaricus, Lactobacillus acidophilus, Lactobacillus lactis, Lactobacillus helveticus. Lactobacillus casei, Lactobacillus reuteri* und *Lactobacillus delbrueckii.*

7. Die Zusammensetzung gemäß Anspruch 6, wobei der *Lactobacillus-*Mikroorganismus *Lactobacillus acidophilus* ist.

8. Die Zusammensetzung gemäß irgendeinem der Ansprüche 1-7, wobei der *Bifidobacterium-*Mikroorganismus ausgewählt wird unter *Bifidobacterium bifidum*, *Bifidobacterium longum* und *Bifidobacterium infantis.*

9. Die Zusammensetzung gemäß Anspruch 8, wobei der *Bifidobacterium-*Mikroorganismus *Bifidobacterium longum* ist.

10. Die Zusammensetzung gemäß irgendeinem der Ansprüche 1-9, wobei die probiotische Komponente mehr als einen *Lactobacillus-* oder *Bifidobacterium-*Mikroorganismus umfasst.

11. Die Zusammensetzung gemäß irgendeinem der Ansprüche 1-9, wobei die probiotische Komponente einen *Lactobacillus*-Mikroorganismus umfasst.

12. Die Zusammensetzung gemäß irgendeinem der Ansprüche 1-9, wobei die probiotische Komponente einen *Bifidobacterium*-Mikroorganismus umfasst.

13. Eine Zusammensetzung gemäß irgendeinem der Ansprüche 1-12 zur Anwendung bei der Reduktion der Blutkonzentration von stickstoffhaltigen Abfallprodukten, die sich im Blutkreislauf anhäufen.

14. Eine Zusammensetzung gemäß irgendeinem der Ansprüche 1-12 zur Anwendung bei der Behandlung von Nierenversagen.

15. Anwendung der Zusammensetzung gemäß irgendeinem der Ansprüche 1-12 zur Herstellung eines Medikaments für die Behandlung von Nierenversagen.

## Revendications

1. Une composition gastro-résistante comprenant au moins un composant probiotique et au moins un composant prébiotique, **caractérisée en ce que** le composant probiotique comprend une culture mixte de bactéries sélectionnées parmi les microorganismes suivants : *Streptococcus, Lactobacillus, Bacillus* et *Bifidobacteria,* et **caractérisée en ce que** les bactéries sont fournies à une concentration de 10¹⁰ cfu/g à 10¹² cfu/g ou de 10¹⁰ cfu/mL à 10¹² cfu/mL.

2. La composition selon la revendication 1, **caractérisée en ce que** le composant probiotique comprend un microorganisme *Lactobacillus* ou *Bifidobacteria.*

3. La composition selon l'une quelconque des revendications 1 à 2, **caractérisée en ce que** le composant prébiotique comprend un oligosaccharide ou une pyrodextrine.

4. La composition selon l'une quelconque des revendications 1 à 3, **caractérisée en ce que** le microorganisme *Streptococcus* est sélectionné parmi les microorganismes suivants : *Streptococcus thermophilus, Streptococcus diacetiliactis, Streptococcus cremoris, Streptococcus durans* et *Streptococcus faecalis.*

5. La composition selon l'une quelconque des revendications 1 à 4, **caractérisée en ce que** le microorganisme *Streptococcus* est *Streptococcus thermophilus.*

6. La composition selon l'une quelconque des revendications 1 à 5, **caractérisée en ce que** le microorganisme *Lactobacillus* est sélectionné parmi les microorganismes suivants : *Lactobacillus bulgaricus, Lactobacillus acidophilus, Lactobacillus lactis, Lactobacillus helveticus, Lactobacillus casei, Lactobacillus reuteri* et *Lactobacillus delbrueckii.*

7. La composition selon la revendication 6, **caractérisée en ce que** le microorganisme *Lactobacillus* est *Lactobacillus acidophilus.*

8. La composition selon l'une quelconque des revendications 1 à 7, **caractérisée en ce que** le microorganisme *Bifidobacteria* est sélectionné parmi les microorganismes suivants : *Bifidobacterium bifidum, Bifidobacterium longum* et *Bifidobacterium infantis.*

9. La composition selon la revendication 8, **caractérisée en ce que** le microorganisme *Bifidobacteria* est *Bifidobacterium longum.*

10. La composition selon l'une quelconque des revendications 1 à 9, **caractérisée en ce que** le composant probiotique comprend plus d'un microorganisme *Lactobacillus* ou *Bifidobacteria.*

11. La composition selon l'une quelconque des revendications 1 à 9, **caractérisée en ce que** le composant probiotique comprend un microorganisme *Lactobacillus.*

12. La composition selon l'une quelconque des revendications 1 à 9, **caractérisée en ce que** le composant probiotique comprend un microorganisme *Bifidobacteria.*

13. Une composition selon l'une quelconque des revendications 1 à 12, destinée à être utilisé pour réduire la concentration sanguine des déchets azotés qui s'accumulent dans la circulation sanguine.

14. Une composition selon l'une quelconque des revendications 1 à 12, destinée à être utilisé pour le traitement de l'insuffisance rénale.

15. Une composition selon l'une quelconque des revendications 1 à 12, destinée à être utilisée pour la fabrication d'un médicament pour le traitement de l'insuffisance rénale.
